# EUROPÄISCHE PATENTSCHRIFT

(11) **EP 1 206 389 B1**
(45) Veröffentlichungstag und Bekanntmachung des Hinweises auf die Patenterteilung: **15.12.2004**
(21) Anmeldenummer: 00954394.3
(22) Anmeldetag: 03.08.2000
(51) Int. Cl.: B65B 55/04

(54) **VERFAHREN UND VORRICHTUNG ZUR STERILEN VERPACKUNG VON STOFFEN IN KUNSTSTOFFBEHÄLTNISSE**
METHOD AND DEVICE FOR STERILE PACKAGING OF SUBSTANCES IN CONTAINERS MADE OF SYNTHETIC MATERIAL
PROCEDE ET DISPOSITIF D'EMBALLAGE STERILE DE SUBSTANCES DANS DES CONTENANTS EN MATIERE SYNTHETIQUE

(30) Priorität: 05.08.1999 DE 19937008
(43) Veröffentlichungstag der Anmeldung: 22.05.2002
(73) Patentinhaber: ROBERT BOSCH GMBH, 70442 Stuttgart (DE)
(72) Erfinder: WILKE, Bernd, D-71397 Leutenbach (DE); WEBER, Thomas, D-70193 Stuttgart (DE); RAUSCHNABEL, Johannes, D-70197 Stuttgart (DE); HENKE, Sascha, D-71263 Weil der Stadt (DE); KLEFENZ, Friedbert, D-61350 Bad Homburg (DE)
(86) Internationale Anmeldenummer: PCT/DE2000/002586
(87) Internationale Veröffentlichungsnummer: WO 2001/010720

(56) Entgegenhaltungen:
- WO-A-98/51608
- DE-A- 4 408 301
- US-A- 5 521 351

## Beschreibung

### Stand der Technik

Die Erfindung betrifft ein Verfahren zur sterilen Verpakkung von Stoffen in Kunststoffbehältnisse, wobei die Stoffe hierbei bevorzugt flüssig sind, und eine Vorrichtungen zur Durchführung des Verfahrens, ausgehend von den gattungsgemäßen Merkmalen der Haupt- und Nebenansprüche.

Es ist hinlänglich bekannt, zur Beseitigung von schädlichen Mikroorganismen oder Keimen in Behältnissen in der Medizin oder der Lebensmitteltechnologie, z.B. bei Ampullen, Schnappdeckelgläser, Septengläser oder sog. Vials physikalische oder chemische Verfahren einzusetzen. Beispielsweise können bei einem Wasserdampfverfahren mit einer wässrigen Vorreinigung die Behältnisse über einen vorgegebenen Zeitraum heißem Wasserdampf ausgesetzt werden. Die Dauer des Prozesses erfordert große Anlagen, um in den Fertigungsfluss integriert hohe Stückzahlen sterilisieren zu können. Die Sterilisation muß vollständig erfolgen, d.h. unter Abtötung sämtlicher Keime. Die sterilisierten Ampullen müssen dabei vor dem Befüllen außerdem getrocknet werden, wodurch mit den dafür benötigten Trocknereinheit das Anlagenvolumen zusätzlich vergrößert wird.

Diese Wasserdampfsterilisation ist jedoch nicht in der Lage sog. pyrogene, d.h. entzündlich wirkende Abbauprodukte und Zellrestbestandteile von abgetöteten Keimen vollständig zu entfernen. Eine Behandlungen beispielsweise mit Wasserstoffperoxid-Dampf führt zwar zur Oxidation von abgetöteten Zellbestandteilen, erfordert aber eine sorgfältige Reinigung und einen dementsprechend hohen Aufwand. Beide Verfahren können bisher nur bei Glasgefäßen angewendet werden, da die notwendigen Temperaturen für Kunststoffgefäße zu hoch sind.

Aus der EP 0 377 788 A1 ist ein Verfahren bekannt, bei dem zur Sterilisation von Gegenständen ein Plasma mit einer elektromagnetischen Strahlung mit einer Frequenz von etwa 2,45 GHz erzeugt wird. Der Gegenstand wird hierzu vollständig einem Niederdruckplasma ausgesetzt und in einer Erweiterung auch mit einer zusätzlichen Wärmequelle bestrahlt. Die Sterilisation mittels eines Plasmas ist bisher im wesentlichen auf die Applikation in der Behandlung medizinischer Geräte konzentriert, zum Beispiel bei Kathetern oder Transfusionsbestecken.

### Vorteile der Erfindung

Ein Verfahren zur Sterilisation von Behältnissen oder Gegenständen wird gemäß der Erfindung mit den Merkmalen des Hauptanspruchs in vorteilhafter Weise derart ausgebildet, dass eine Plasmabehandlung, z.B. eine Plasmasterilisation, durchgeführt wird, bei der ein räumlich und/oder zeitlich selektives Anregen des Plasmas in verschiedenen Bereichen der Wände des Behältnisses, vorgenommen wird. Mit dem erfindungsgemäßen Verfahren und einer Vorrichtung zur Durchführung des Verfahrens ist eine aseptische, im Falle von Pharmaprodukten sogar eine pyrogenfreie Verpakkung von Flüssigkeiten in Kunststoffbehältern, wie Ampullen, Flaschen oder Vials in vorteilhafter Weise durchführbar und mit weiteren Bearbeitungsschritten kombinierbar geworden.

Die Plasmasterilisation ermöglicht auf einfache Weise mit sehr kurzen Prozeßzeiten gleichzeitig die Abtötung von Keimen und die vollständige Beseitigung von Pyrogenen, im Inneren, wie auf der Außenwand der Behältnisse. Die sterilen Behältnisse sind dann frei von vermehrungsfähigen keimen; eine abschließende Trocknung der Behältnisse ist nicht notwendig. Die beschriebene erfindungsgemäße Plasmabehandlung der Behältnisse ist ein relativ kaltes Verfahren, mit dem auch temperaturempfindliche Kunststoffe wie Polyethylen, Polypropylen oder PVC behandelt werden können.

Die Erfindung ermöglicht es in vorteilhafter Weise, dass mit dem Verfahren auch eine Beschichtung gegen Sauerstoffpermeation der Behältniswände aus Kunststoff zusätzlich zur Sterilisation bzw. Entpyrogenisierung vorgenommen werden kann. Hierbei kommen beispielsweise die sog. PVD-Verfahren in Form einer Dampfabscheidung mit physikalischen Methoden (z.B. Magnetzerstäubung) oder PECVD-Verfahren in Form einer plasmaunterstützten Dampfabscheidung, bei der eine chemische Reaktion stattfindet (z.B. Plasmapolymerisation) zur Anwendung.

In eine einzige Vorrichtung kann somit eine Plasmabeschichtung, vorzugsweise durch Aufbringen einer Plasmapolymerschicht, integriert werden, die die Innenwände des Kunststoffbehältnisses vor der Befüllung gegen Permeation von Sauerstoff und Wasserdampf und gegen die Migration von Weichmachern und Lösungsmitteln aus dem Kunststoff in die zu verpackende Flüssigkeit schützt. In besonders vorteilhafter Weise kann diese Plasmabeschichtung mit der Plasmasterilisation identisch sein, d.h. während der Beschichtung findet durch das Beschichtungsplasma selbst gleich auch die Zerstörung von Keimen/Pyrogenen, bzw. deren Überdeckung durch die Polymerschicht, statt.

Ein weiterer Behandlungsschritt kann in vorteilhafter Weise in einer Plasmaaktivierung, vorzugsweise der Außenseite des Behältnisses, bestehen, der auch gleichzeitg mit der Plasmasterilisierung in einem Prozesszyklus durchgeführt werden kann.

Mit dem erfindungsgemäßen Verfahren ist es darüber hinaus möglich, dass sogar die Herstellung der Behälter zu Beginn des Bearbeitungsvorganges, z.B. durch Spritzguss, durch Spritzblasformen, durch Verschweißen von Kunststofffolien oder durch Tiefziehen in die Vorrichtung integriert wird. Möglich ist hier auch eine Herstellung durch Extrusion, insbesondere durch Extrusionsblasformen oder ähnliches Verfahren.

Auch eine Befülleinrichtung am Ende der Verfahrensschritte kann sich die Tatsache zunutze machen, dass die Behältnisse nach der Sterilisation oder Entpyrogenisierung unter Vakuum stehen und somit steril und ohne aufwendige Restentlüftung befüllt werden können. Es lässt sich also prinzipiell die Herstellung der Kunststoffbehältnisse, eine Behälterinnenbeschichtung, eine Sterilisation, eine Befüllung und auch noch die Versiegelung der Behältnisse in einer Vorrichtung realisieren, die vollständig aspetisch gegenüber der Umgebung verkapselbar ist.

Bevorzugte Verfahrensschritte zur Durchführung der Plasmasterilisation werden mit den Unteransprüchen 12 bis 15 vorgeschlagen, die mit den weiteren oben erwähnten Behandlungsschritten vorteilhaft kombinierbar sind und für sich gesehen in der nichtvorveröffentlichten DE 199 03 935.6 beschrieben sind.

Eine vorteilhafte Vorrichtung zur Durchführung eines der oben beschriebenen Verfahren ist im Anspruch 16 und den darauf rückbezogenen Ansprüchen angegeben, in der eine Verkettung der beschriebenen Bearbeitungsvorgänge in einer Anlage möglich ist. Es ergeben sich hierbei zusammenfassend folgende Vorteile:

Durch die Kapselung in der Vorrichtung können aseptische Verpackungsvorgänge für Behältnisse oder Behälter in verhältnismäßig kontaminierter äußerer Umgebung durchgeführt werden.

Da die für die Plasmaanregung erforderliche Vakuumtechnik relativ hohe Investitionskosten verursacht, ist eine Verkettung von mehreren, bisher räumlich getrennten Bearbeitungssschritten in einer Vakuumanlage kostengünstig, da die Anlage maximal ausgenutzt wird. Mit der evakuierbaren Kammer der Vakuumanlage kann somit eine Verkettung mehrerer der oben beschriebenen Herstellungs- oder Behandlungsschritte in einer Prozesslinie vorgenommen werden, wobei sich dabei das Vakuum beispielsweise auch nur im jeweiligen Behältnis befinden kann.

Die Plasmasterilisation, die Plasmapolymerisation und die Plasmaaktivierung sind Prozesse, bei denen sparsam mit den gasförmigen Medien umgegangen wird und die in der Regel mit ungiftigen, aus Umweltgesichtspunkten unkritischen Stoffen durchgeführt werden können.

Bei einer integrierten Herstellung der Behältnisse mittels einer Kunststoffverarbeitung kann die Vorreinigung herantransportierter Behältnisse mit den dazu notwendigen Waschmedien und die Entsorgung der kontaminierten Medien entfallen.

Die Sterilisation bzw. Entpyrogenisierung der Behältnisse führt zu relativ unkritischen Abbauprodukten, wie CO, CO₂, CH₄, H₂O oder zu kurzkettigen Aliphaten.

Der Ersatz von in herkömmlicher Weise verwendeten Glasgefäßen durch Kunststoffbehältnisse in den erwähnten Anwendungsgebieten führt insbesondere bei aseptischen Pharmaverpackung zu großen Produktivitäts- und Kostenvorteilen.

Weiterhin ist Gefährdung der Mitarbeiter durch bei Havariefällen oder Fehlbedienung austretende heiße oder gesundheitsschädliche Medien in der hier beanspruchten vakuumtechnik weitgehend ausgeschlossen.

Diese und weitere Merkmale von bevorzugten Weiterbildungen der Erfindung gehen außer aus den Ansprüchen, einschließlich der rückbezogenen Unteransprüche, auch aus der Beschreibung und den Zeichnungen hervor, wobei die einzelnen Merkmale jeweils für sich allein oder zu mehreren in Form von Unterkombinationen bei der Ausführungsform der Erfindung und auf anderen Gebieten verwirklicht sein und vorteilhafte sowie für sich schutzfähige Ausführungen darstellen können, für die hier Schutz beansprucht wird.

### Zeichnung

Ausführungsbeispiele von Vorrichtungen zur Durchführung des erfindungsgemäßen Verfahrens zur Behandlung, insbesondere zur Plasmasterilisation und Weiterbehandlung von Behältnissen werden anhand der Zeichnung erläutert. Es zeigen:
Figur 1 eine schematische Ansicht einer Vorrichtung mit einem in einer Kammer angeordneten Behältnis, wobei in der Kammer ein Vakuum erzeugt wird;
Figur 2 eine schematische Ansicht einer Vorrichtung mit einem in einer Kammer angeordneten Behältnis, wobei im Behältnis ein Vakuum erzeugt wird und
Figur 3 eine schematische Ansicht eines Verfahrensablaufs zur Durchführung des erfindungsgemäßen Verfahrens.

### Beschreibung der Ausführungsbeispiele

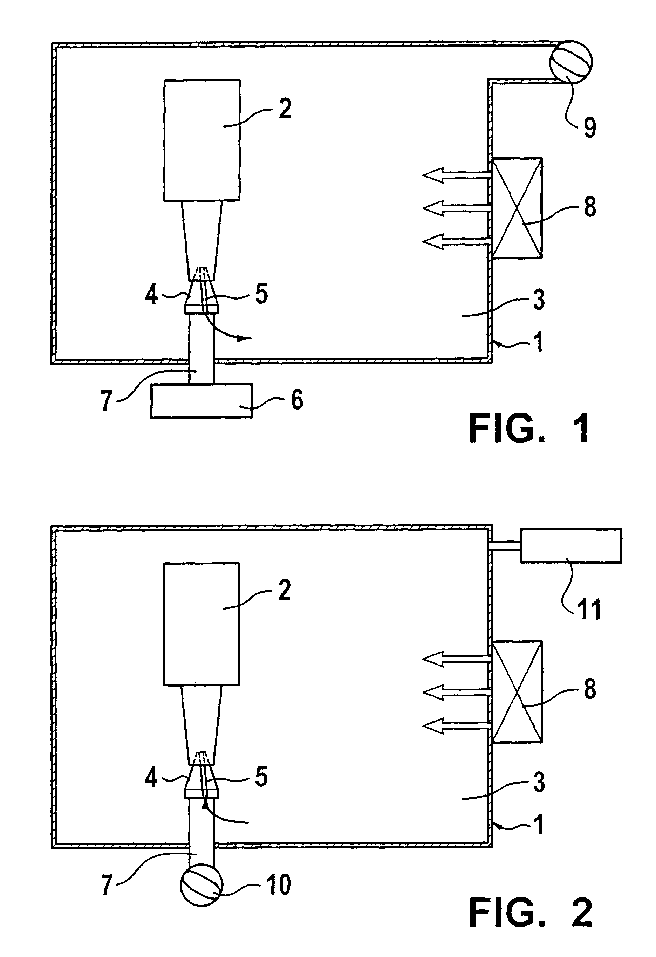

In Figur 1 ist ein erstes Ausführungsbeispiel einer Vorrichtung 1 dargestellt, an der hier zunächst eine Plasmasterilisierung von Behältnissen 2 erläutert wird. In einer gegen die umgebende Atmosphäre abschließbaren, evakuierbaren Kammer 3 (Vakuumkammer) erfolgt der Sterilisationsprozess, wobei das Behältnis 2 beim dargestellten Ausführungsbeispiel auf einem Konus 4 als Behältnishalterung gehalten wird. Der Konus 4 weist außen eine Leckage-Nut 5 auf, die somit zwischen dem inneren Rand der Öffnung des aufliegenden Behältnisses 2 und der Außenwand des Konusses 4 liegt und eine, wenn auch geringe, Gasströmung zwischen der Kammer 3 und dem Inneren des Behältnisses 2 ermöglicht. Durch den innen offenen Konus 4 ist über eine Zuleitung 7 von außerhalb der Kammer 3, gesteuert mit einem Durchflussregler 6, ein Gas in das Behältnis 2 zuführbar. Außen an der Kammer 3 ist stilisiert eine Plasmaquelle 8, z.B. zur Erzeugung elektromagnetischer Wechselfelder, gezeigt und es ist weiterhin eine Pumpe 9 zur Absaugung von Gas aus der Kammer 3 vorhanden.

Das Prinzip des hier angewendeten Sterilisationsverfahrens beruht auf einem an sich bekannten physikalischen Prozess, der zur Erzeugung eines Plasmas aus einem permanenten Gas führt, wobei die Atome des Gases durch eine geeignete Energiezufuhr in ein Gemisch von Elektronen und Ionen umgewandelt werden. Die Energiezufuhr erfolgt hier bevorzugt durch Stöße zwischen Teilchen aufgrund der Beschleunigung der Ladungsträger der Elementarteilchen, insbesondere der Elektronen, in elektrischen Feldern, die dem Plasma von außen mit der Plasmaquelle 8 aufgeprägt werden.

Gemäß des erfindungsgemäßen Ausführungsbeispiels nach der Figur 1 und bei einem weiter unten beschriebenen zweiten Ausführungsbeispiel nach Figur 2 erfolgt dabei eine selektives Anregung des Plasmas. Durch zumindest zeitweise während der Verfahrensschritte hervorgerufene unterschiedliche Gasdrücke im Inneren des Behältnisses 2 und in der umgebenden Kammer 3 kann das Plasma gezielt entweder innen oder außen angeregt werden. Bei einem zu geringem Druck können nicht genug Elementarteilchen angeregt bzw. ionisiert werden um eine Plasmaentladung aufrecht zu erhalten. Bei einem zu hohem Druck des Gases ist die mittlere freie Weglänge zu gering, um den Elementarteilchen zwischen zwei Stößen genug Beschleunigungsstrecke oder Beschleunigungszeit zur Aktivierung oder Anregung und damit zur Ionisation zu lassen.

Beim ersten Ausführungsbeispiel nach der Figur 1 wird die Kammer 3 mit der Pumpe 9 so abgepumpt, daß der Gasdruck in ihrem Inneren der Kammer 3 für die Anregung eines Plasmas zu gering ist. In der Kammer 3 sind die zu sterilisierenden Behältnisse 2 so gehalten, dass sie direkt mit ihrer Öffnung formschlüssig auf dem Konus 4 sitzen. Über die Zuleitung 7 strömt von außerhalb der Kammer 3, gesteuert durch den Durchflussregler 6, ein definierter Gasstrom aus beispielsweise Sauerstoff, gefilterte Luft, Wasserdampf, Wasserstoffperoxid-Dampf, Argon, Stickstoff, Tetrafluormethan, Schwefelhexafluorid o.ä., durch das Innere des Konusses 4 in das Behältnis 2. Dieser Gasstrom ist so eingestellt, dass im Inneren des Behältnisses 2 der Druck so groß wird, dass ein Plasma angeregt werden kann.

Um einen gewünschten Druckgradienten bzw. bei einem bestimmten Druck einen definierten Gasaustausch im Behältnis 2 nach der Figur 1 zu erhalten, wird über die Leckage-Nut 5 außen am Konus 4 das Gas, bzw. das Abgas des Plasmas, aus dem Behältnis 2 in die Kammer 3 abgesaugt. In Abhängigkeit von der durch den Konus 4 zuströmenden Gasmenge und dem Leitwert der Leckage-Nut 5 ist dabei der Druck des Gases im Behältnis 2 einstellbar. Möglich ist hier auch eine gesteuerte Leckage-Nut, beispielsweise mit einem Ventil. Das in die Kammer 3 durch die Leckage-Nut 5 zugeströmte Abgas wird dann mit der Pumpe 9 zur Erhaltung der Druckverhältnisse aus der Kammer 3 abgesaugt, wodurch somit ein Plasma selektiv nur im Inneren des zu sterilisierenden Behältnisses 2 erzeugt ist.

In einer besonders vorteilhaften weitergebildeten Ausführung dieses selektiven Sterilisationsprozesses wird die Kammer 3 nach der Figur 1 zuerst vollständig abgepumpt ohne einen Gasfluss in das Behältnis 2 oder die Kammer 3 einströmen zu lassen. Anschließend wird der Durchflussregler 6 zur Erzeugung eines definierten Gasstroms geöffnet, der in das zu sterilisierenden Behältnis 2 einfließt und im Inneren des Behältnisses 2 ein Plasma zündet, vorteilhafterweise durch Mikrowelleneinstrahlung der Plasmaquelle 8.

Wenn die gewünschte Sterilisationswirkung im Inneren des Behältnisses 2 erreicht ist, kann optional noch in der Kammer 3, also außerhalb des zu sterilisierenden Behältnisses 2, der Gasdruck erhöht werden, indem ein definierter Gasstrom desselben Gases, bzw. Gasgemisches, das durch die Zuleitung 7 fließt, oder gegebenenfalls auch durch einen zusätzlichen, hier nicht dargestellten Einlass in die Kammer 3 eingelassen wird, so dass ein Plasma auch hier angeregt werden kann. Das Plasma erlischt dabei im Inneren des Behältnisses 2 und wird außerhalb in der Kammer 3 angeregt. Mit diesem Verfahrensschritt lässt sich somit das Behältnis 2 auch an der, der Kammer 3 zugewandten Außenwand sterilisieren.

Dieser zuvor beschriebene Vorgang des nach außen Springens der Plasmabildung entsteht dadurch, dass ein Plasma sich nach außen abschirmt, d.h. die eingestrahlte Energie soweit absorbiert, dass die Energie außerhalb dieses Plasmas nicht ausreicht, um ein weiteres Plasma anzuregen. So schirmt das äußere Plasma in der Kammer 3 sich gegenüber der abgetrennten Gasatmosphäre im Inneren des zu sterilisierenden Behältnisses 2 ab und verhindert dort die Anregung eines Plasmas. Ist der Druck dabei in der Kammer 3 zu gering, aber im Inneren des Behältnisses 2 für ein Plasma ausreichend, so wird die eingestrahlte Energie im inneren Plasma des Behältnisses 2 weitgehend absorbiert. Da die Energie der Plasmaquelle 8 aber zuerst in die Kammer 3 eingestrahlt wird und erst dann durch die Wand des zu sterilisierenden Behältnisses 2, unter Umständen gedämpft, in das Innere des Behältnisses 2 gelangt, wird im anderen Fall das Plasma sofort außen am Behältnis 2 entstehen, wenn in der Kammer 3 der Gasdruck dafür ausreichend ist.

Bei dem zweiten Ausführungsbeispiel nach Figur 2 wird das anhand der Figur 1 beschriebene Prinzip umgekehrt; die im wesentlichen gleichwirkenden Bauteile sind jedoch mit den gleichen Bezugszeichen wie bei der Figur 1 versehen. Das zu sterilisierende Behältnis 2 wird hier direkt über den Konus 4 mit einer Pumpe 10 zur Bildung eines Vakuums im Behältnis 2 abgesaugt. Die Durchführung des Sterilisationsverfahrens mit diesem Ausführungsbeispiel erfolgt nun in der Weise, dass nachdem die Kammer 3 mit dem Sterilisationsgas, bzw. -gasgemisch durch eine Gaszufuhr 11 geflutet ist, das zu sterilisierenden Behältnis 2 soweit abgepumpt wird, dass ein Plasma durch Einstrahlung eines elektromagnetischen Feldes, vorteilhafterweise eines Mikrowellenfeldes, im Inneren des Behältnisses 2 angeregt ist, während der Druck außerhalb des zu sterilisierenden Behältnisses 2 in der Kammer 3 für ein Plasma zu hoch ist (vgl. die Erläuterung weiter oben). Das Gas strömt dabei durch die Leckage-Nut 5 am Konus 4 der Behältnishalterung in das Behältnis 2 ein. Das Abgas im Behältnis 2 kann nunmehr durch den Konus 4 und die Zuleitung 7 mit der Pumpe 10 abgesaugt werden.

In einem anschließenden Verfahrensschritt wird die Kammer 3 zur Sterilisation der Außenflächen des Behältnisses 2 soweit abgesaugt, daß das Plasma außen in der Kammer 3 brennt. Dies kann dadurch erfolgen, daß die Frischgaszufuhr mit dem Durchflussregler der Gaszufuhr 11 gestoppt wird und das Gas aus der Kammer 3 über die Leckage-Nut 5 am Konus 4 nach und nach abgesaugt wird bis der entsprechende Druck in der Kammer 3 erreicht ist. Alternativ ist dies auch mit hier nicht dargestellten Pumpen zu bewerkstelligen, mit denen die Kammer 3 zusätzlich evakuiert werden kann.

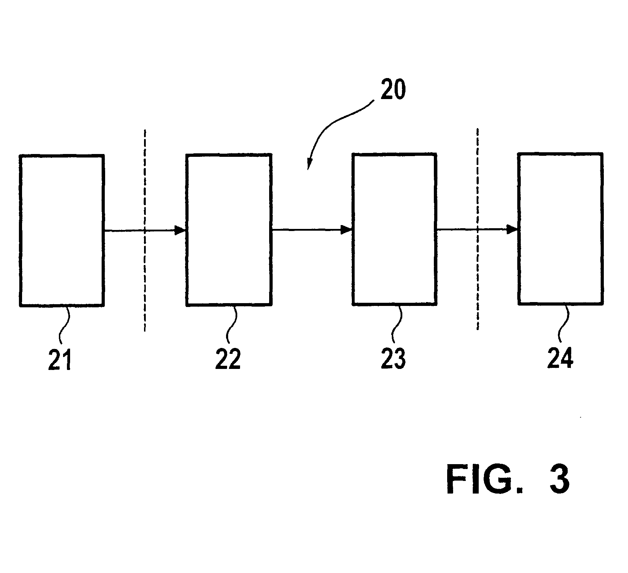

Mit den zuvor beschriebenen Ausführungsbeispielen ist eine Sterilisation von einzelnen Behältnissen in einer relativ einfachen Vorrichtung erläutert worden. Die beschriebenen Vorgehensweisen können auch für die weiteren Plasmabehandlungsschritte Anwendung finden. Anhand eines in Figur 3 dargestellten Ablaufdiagramms sollen Herstellungs- und Behandlungsschritte in einer schematisch angedeuteten Anlage 20 erläutert werden, die infolge einer Verkettung der Schritte einen räumlich und zeitlich eng beieinander liegenden Herstellungs- und Bearbeitungsprozess der Behältnisse 2 ermöglicht. Kern der Anlage 20 ist die bei den Figuren 1 und 2 erläuterte evakuierbare Kammer 3, in der mit einer entsprechenden konstruktiven Erweiterung z.B. ein Herstellungsschritt 21, ein Sterilisierungs und/oder Beschichtungsschritt 22, ein Akt ivierungsschritt 23 und ein Befüllungs- bzw. Versiegelungsschritt 24 durchgeführt werden kann.

In allen Schritten 21 bis 24 kann mit einem in der Kammer 3 hergestellten Vakuum gearbeitet werden; bei dem Herstellungsschritt 21 und bei dem Befüllungs- oder Versiegelungsschritt 24 müssen hierbei jedoch nicht die gleichen Vakuumbedingungen herrschen wie bei den Schritten 22 und 23, wobei hier auch eine Behandlung ohne Vakuum denkbar ist.

Im Herstellungsschritt 21 wird die Herstellung der Behältnisse 2 durch Spritzgießen, durch Spritzblasformen, durch Tiefziehen, durch Verschweißen von Kunststoffolien oder durch Extrusionsblasformen oder ähnliche Verfahren vorgenommen, wobei beispielsweise dass Kunststoffgranulat oder eine Kunststofffolie direkt in die Kammer 3 gebracht wird. Der Schritt 21 kann somit direkt in der Kammer 3 geschehen oder in einem eng mit der Kammer 3 in der Anlage 20 gekoppelten Anlagenbaustein erfolgen. Je nach der Enge der Kopplung, beispielsweise bei einer Herstellung unter den gleichen Vakuumbedingungen wie bei der Plasmasterilisation nach Schritt 22, kann hier sogar eine Herstellung und Sterilisation in einem Schritt erfolgen, bzw. eine separate Sterilisation ganz überflüssig werden, da die für die Behältnisformung notwendigen Temperaturen bereits eine keimabtötende Wirkung haben können.

Die Behandlungsschritte 22 und 23 unter Plasmabedingungen des Vakuums in der Kammer 3 können dann noch eine Beschichtung und/oder die Modifikation der Oberfläche der Behältnisse sowie eine Aktivierung insbesondere der äußeren Oberfläche des Behältnisses 2 beinhalten. Aufgabe der Beschichtung, insbesondere im Inneren, ist es beispielsweise die Permeation von Wasserdampf von der Behältnisumgebung in das Behältnisinnere, die Permeation von Sauerstoff von der Behältnisumgebung in das Behältnisinnere, die Permeation der eingefüllten Flüssigkeit durch die Behältniswand nach außen oder die Migration von Bestandteilen des Behältermaterials, wie Gleitmittel, Stabilisatoren, Weichmacher, Lösungsmittel oder dergleichen, in die in das Behältnis 2 eingefüllt Flüssigkeit zu verhindern.

Die Beschichtung kann durch die Deposition einer dünnen, z.B. 10-1000 nm dicken, Plasmapolymerschicht geschehen, die im Plasma mittels Einstrahlung des oben beschriebenen elektromagnetischen Feldes, z.B. im Bereich von 100 KHz bis 4,9 GHz, aus einem silicium- und/oder kohlenstoffhaltigen Monomer ( z.B. Silane, Siloxane, Silazane, Aromaten, Alkane, Alkene oder Alkine) entsteht. Diese Beschichtung kann dabei bereits sterilisierende, bzw. pyrogenzerstörende Wirkung haben, insbesondere, wenn das Monomer Sauerstoff enthält. Andererseits können aber auch Sauerstoff, Argon oder Stickstoff bzw. Wasserdampf oder Wasserstoffperoxid als nichtbeschichtendes Gas dem Polymerisationsplasma hinzugefügt werden.

Es ist mit der vorgeschlagenen Vorrichtung somit möglich, dass eine Vakuumbeschichtung in den Takt einer Fertigungslinie für die Herstellung und Bearbeitung von Kunststoffbehältnissen eingebunden werden kann, d.h. der Spritzguss der Thermoplastgegenstände findet im gleichen Takt wie deren weitere Bearbeitungsschritte, wie z.B. die Plasmasterilisation und gegebenenfalls Plasmapolymerisation statt. Damit können zahlreiche logistische Probleme, die bei der herkömmlichen Befüllung z.B. von Glasampullen existieren, entfallen, wie Herstellung der Glasampullen in einem Glaswerk, Transport zum Pharmawerk, Vorreinigung/Waschen und Bestückung der Anlage.

Zusätzlich zur Beschichtung, Sterilisation und Entpyrogenisierung der Innenseite der Behältnisse 2 kann auch, wie zuvor erwähnt, in einem Behandlungsschritt 23 zusätzlich die Außenseite der Behältnisse 2 mittels einer Plasmaaktivierung, auch hier im Bereich von 100 KHz bis 4,9 GHz, mit nichtbeschichtenden Gasen (Ar, He, Ne, O₂, N₂) für das äußere Bedrucken oder Verkleben/Versiegeln der Behältnisse 2 in der Anlage 20 nach der Figur 3 vorbereitet werden. Diese Aktivierung der Kunststoffoberfläche kann allerdings ev. auch schon durch die vorhergehenden Plasmabehandlungsschritte mitgeleistet werden.

Bei der anhand der Figur 3 beschriebenen Anlage 20 ist es dann abschließen auch noch möglich, die endgültige Versiegelung der im Behandlungsschritt 24 durchgeführten Befüllung der Behältnisse 2 durchzuführen. Die Befüllung der Behältnisse 2 mit der Anlage 20 nach der Figur 3 kann leicht in den Behandlungsprozess integriert werden, da in den Behältnissen 2 aufgrund des Vakuums keine Restluft verdrängt werden muss. Dies ist insbesondere bei pharmazeutischen Behältnissen von großem Vorteil, da hier keine kontaminierte, d.h. keim-, bzw. pyrogenhaltige Luft zwischen den Schritten der Sterilisation und der Befüllung in die Behältnisse 2 geraten kann und die Restluftabsaugung während der Befüllung entfällt, gegebenenfalls kann die Befüllung unter Schutzgas (N₂, Ar, SF₆) erfolgen.

Die Versiegelung oder Verschweissung der Behältnisse 2 läßt sich bei der Anlage nach der Figur 3 auch in der Vakuumkammer durchführen, so dass erst das fertig befüllte und versiegelte Behältnisse 2 ausgeschleust werden muss. Eine kontaminierte Umgebungsluft kann daher nicht in das Behältnis 2 gelangen. Insgesamt ist somit insbesondere bei Verwendung von Kunststoffbehältern eine Behältnisherstellung und Weiterverarbeitung vor Ort und eine optimale Ausnutzung des Vakuums im Anlagenbereich bei Anwendung einer Plasmabehandlung der Behältnisse 2 möglich.

## Patentansprüche

1. Verfahren zur Behandlung und/oder Herstellung von Kunststoffbehältnissen (2), bei dem
- in mindestens einem Verfahrensschritt in einer Kammer (3) eine Plasmabehandlung durch Anregung einer elektromagnetischen Schwingung derart durchgeführt wird, dass das Plasma in einem Vakuum räumlich und/oder zeitlich selektiv angeregt wird und an Wänden der Behältnisse (2) anliegt und
- unter Ausnutzung des Vakuums weitere Herstellungsund/oder Behandlungsschritte (21,22,23,24) an oder mit den Kunststoffbehältnissen (2) derart durchgeführt werden, dass diese während des Herstellungs- oder Behandlungsprozesses zur sterilen Verpackung von Stoffen in den Kunststoffbehältnissen (2) miteinander verkettet sind.

2. Verfahren nach Anspruch 1, **dadurch gekennzeichnet, dass**
- die Plasmabehandlung eine Sterilisation und/oder eine Entpyrogenisierung ist.

3. Verfahren nach Anspruch 1 oder 2, **dadurch gekennzeichnet, dass**
- ein weiterer Behandlungsschritt (22) in einer Permeationssperrbeschichtung, vorzugsweise der Innenwände der Behältnisse (2) besteht.

4. Verfahren nach Anspruch 3, **dadurch gekennzeichnet, dass**
- die Beschichtung durch Aufbringen einer Plasmapolymerschicht erfolgt.

5. Verfahren nach Anspruch 1 oder 2, **dadurch gekennzeichnet, dass**
- zumindest Bereiche der Oberfläche der Behältnisse (2) durch eine Plasmabehandlung chemisch so verändert wird, dass die Permeation von Stoffen durch die Behälterwand erschwert wird.

6. Verfahren nach einem der Ansprüche 3 bis 5, **dadurch gekennzeichnet, dass**
- die Plasmabehandlung sowie die Beschichtung und/oder die Modifikation der Oberfläche der Behältnisse (2) als mindestens ein weiterer Behandlungsschritt in einem Prozesszyklus in der Kammer erfolgt.

7. Verfahren nach einem der vorhergehenden Ansprüche, **dadurch gekennzeichnet, dass**
- während oder nach der Plasmabehandlung eine Plasmaaktivierung (23), vorzugsweise der Außenseite der Behältnisse (2) erfolgt.

8. Verfahren nach einem der vorhergehenden Ansprüche, **dadurch gekennzeichnet, dass**
- die Herstellung (21) der Behältnisse (2) vor oder in der Kammer, vorzugsweise unter Ausnutzung des Vakuums, vor den weiteren Behandlungsschritten (22,23,24) erfolgt.

9. Verfahren nach Anspruch 8, **dadurch gekennzeichnet, dass**
- die Herstellung (21) der Behältnisse (2) in der Kammer (3) durch Spritzgießen, durch Tiefziehen, durch Verschweißen von Kunststofffolien oder durch Blasextrusion oder ähnliche Verfahren erfolgt.

10. Verfahren nach einem der vorhergehenden Ansprüche, **dadurch gekennzeichnet, dass**
- einer der weiteren Behandlungsschritte (24) die Befüllung der Behältnisse (2)ist.

11. Verfahren nach einem der vorhergehenden Ansprüche, **dadurch gekennzeichnet, dass**
- einer der weiteren Behandlungsschritte (24) die Verschließung und/oder die Versiegelung der Behältnisse (2) ist.

12. Verfahren nach einem der vorhergehenden Ansprüche, **dadurch gekennzeichnet, dass**
- die Behältnisse (2) zur Durchführung der Plasmabehandlung in die Kammer (3), in der zumindest nahezu und in Teilbereichen ein Vakuum herstellbar ist, geführt werden oder in dieser verbleiben, dass
- in das Innere der Behältnisse (2) über eine von der Kammer (3) abgeschirmte Zuleitung (7) ein zur Anregung eines Plasmas geeignetes Gas geführt wird, wobei ein Gasdruckgradient im Inneren so eingestellt und gehalten wird, dass hier ein Plasma angeregt und eine vorgegebene Zeit aufrechterhalten wird und dass
- der Gasdruckgradient und das Plasma im Inneren der Behältnisse (2) durch eine ausreichende Höhe des Druckwertes gegenüber dem Druckwert in der Kammer (3) auch mit einem vorgegebenen Abfluss des Abgases aus den Behältnissen (2) in die Kammer (3) und einer anschließenden Absaugung aus der Kammer (3) aufrecht erhalten wird.

13. Verfahren nach Anspruch 12, **dadurch gekennzeichnet, dass**
- in einem ersten Verfahrensschritt die Kammer (3) evakuiert wird,
- in einem zweiten Verfahrensschritt das Gas in die Behältnisse (2) zur Anregung des Plasmas im Inneren eingeleitet wird, dass
- in einem dritten Verfahrensschritt ein Gas zur Anregung eines Plasmas in die Kammer (3) geleitet wird, zur Anregung eines Plasmas außen an den Behältnissen (2) bei gleichzeitigen Erlöschen des Plasmas im Inneren des Behältnisses (2).

14. Verfahren nach einem der Ansprüche 1 bis 11, **dadurch gekennzeichnet, dass**
- die Behältnisse (2) zur Durchführung der Plasmasterilisation in eine Kammer (3) geführt werden oder in dieser verbleiben, in die ein zur Anregung eines Plasmas geeignetes Gas geführt wird, dass
- im Inneren der Behältnisse (2) über eine von der Kammer (3) abgeschirmte Zuleitung (7) eine zumindest teilweise Evakuierung herstellbar ist wobei ein Gasdruckgradient im Inneren so eingestellt und gehalten wird, dass hier ein Plasma angeregt und eine vorgegebene Zeit aufrechterhalten wird und dass
- der Gasdruckgradient und das Plasma im Inneren der Behältnisse (2) durch eine ausreichende Tiefe des Druckwertes gegenüber dem Druckwert in der Kammer (3) auch mit einem vorgegebenen Zufluss des Gases aus der Kammer (3) in die Behältnisse (2) und einer anschließenden Absaugung aus den Behältnissen (2)aufrecht erhalten wird.

15. Verfahren nach Anspruch 14, **dadurch gekennzeichnet, dass**
- in einem ersten Verfahrensschritt die Kammer (3) mit dem Gas versorgt wird, dass
- in einem zweiten Verfahrensschritt die Behältnisse (2) soweit evakuiert werden, dass über den Zufluss des Gases aus der Kammer (3) die Anregung des Plasmas im Inneren erfolgt und dass
- in einem dritten Verfahrensschritt die Gaszufuhr in die Kammer (3) gestoppt wird, zur Anregung eines Plasmas in der Kammer (3) und damit außen an den Behältnissen (2) bei gleichzeitigen Erlöschen des Plasmas im Inneren des Behältnisses (2).

16. Vorrichtung zur Durchführung des Verfahren nach einem der vorhergehenden Ansprüche, **dadurch gekennzeichnet, dass**
- die zumindest teilweise evakuierbare Kammer (3) Bestandteil einer Anlage (20) zur Herstellung und/oder Behandlung von Behältnissen (2) ist, in der eine Verkettung der Herstellungs- und/oder Behandlungsschritte (21,22,23,24) in mindestens einem Prozesszyklus durchführbar ist.

17. Vorrichtung nach Anspruch 16, **dadurch gekennzeichnet, dass**
- in oder an der Kammer (3) eine Spritzgusseinrichtung vorhanden ist, zur Herstellung mindestens eines Behältnisses (2).

18. Vorrichtung nach Anspruch 16 oder 17, **dadurch gekennzeichnet, dass**
- in oder an der Kammer (3) eine Befüll- und/oder eine Verschließeinrichtung vorhanden ist, mittels der die Behältnisse (2) mit einem Medium befüll- und gegebenenfalls verschließ- und versiegelbar sind.

19. Vorrichtung nach einem der Ansprüche 16 bis 18 , insbesondere zur Durchführung des Verfahren nach einem der Ansprüche 12 bis 15, **dadurch gekennzeichnet, dass**
- in der Kammer (3) mindestens eine Behältnishalterung vorhanden ist, auf der die Behältnisse (2) nahezu druckdicht mit einer vorgegebenen Leckage gehalten sind, dass
- an die Kammer (3) mindestens eine Evakuierungspumpe (9) und/oder eine Gaszufuhr (11) zur Einleitung der nicht beschichtenden und/oder beschichtenden Gase sowie eine Befüllvorrichtung für die Behältnisse (2) angeschlossen ist und dass
- außen an der Kammer (3) eine Plasmaquelle (8) angebracht ist.

## Claims

1. Method for treating and/or producing plastic containers (2), in which
- in at least one method step, a plasma treatment is carried out in a chamber (3) by inducing an electromagnetic oscillation in such a way that the plasma in a vacuum is excited selectively in terms of space and/or time and is adjacent to the walls of the containers (2) and
- using the vacuum, further production and/or treatment steps (21, 22, 23, 24) are carried out on or with the plastic containers (2) in such a way that they are linked to one another during the production or treatment process for the sterile packaging of substances in the plastic containers (2).

2. Method according to Claim 1, **characterized in that**
- the plasma treatment is a sterilization and/or depyrogenation.

3. Method according to Claim 1 or 2, **characterized in that**
- a further treatment step (22) comprises a permeation barrier coating, preferably of the inner walls of the containers (2).

4. Method according to Claim 3, **characterized in that**
- the coating takes place by applying a plasma polymer layer.

5. Method according to Claim 1 or 2, **characterized in that**
- at least regions of the surface of the containers (2) are chemically changed by a plasma treatment in such a way that the permeation of substances through the container wall is made more difficult.

6. Method according to one of Claims 3 to 5,
**characterized in that**
- the plasma treatment and the coating and/or the modification of the surface of the containers (2) takes place as at least one further treatment step in a process cycle in the chamber.

7. Method according to one of the preceding claims, **characterized in that**
- during or after the plasma treatment, a plasma activation (23) takes place, preferably of the outer side of the containers (2).

8. Method according to one of the preceding claims, **characterized in that**
- the production (21) of the containers (2) takes place before or in the chamber, preferably using the vacuum, before the further treatment steps (22, 23, 24).

9. Method according to Claim 8, **characterized in that** the production (21) of the containers (2) in the chamber (3) takes place by injection moulding, by thermoforming, by welding of plastic films or by extrusion blow-moulding or similar processes.

10. Method according to one of the preceding claims, **characterized in that**
- one of the further treatment steps (24) is the filling of the containers (2).

11. Method according to one of the preceding claims, **characterized in that**
- one of the further treatment steps (24) is the closing and/or sealing of the containers (2).

12. Method according to one of the preceding claims, **characterized in that**
- for carrying out the plasma treatment, the containers (2) are introduced into the chamber (3), in which a vacuum can be established at least virtually and in partial regions, or remain in the said chamber, **in that**
- a gas suitable for exciting a plasma is introduced into the interior of the containers (2) via a feed line (7) shielded from the chamber (3), a gas pressure gradiant being set and maintained in the interior in such a way that a plasma is excited there and maintained for a predetermined time, and **in that**
- the gas pressure gradient and the plasma in the interior of the containers (2) are maintained by an adequately high level of the pressure value in comparison with the pressure value in the chamber (3) even with a predetermined outflow of the exhaust gas from the containers (2) into the chamber (3) and subsequent suction removal from the chamber (3).

13. Method according to Claim 12, **characterized in that**
- in a first method step, the chamber (3) is evacuated,
- in a second method step, the gas is introduced into the containers (2) for exciting the plasma in the interior, and
- in a third method step, a gas for exciting a plasma is introduced into the chamber (3), to excite a plasma on the outside of the containers (2) while at the same time extinguishing the plasma in the interior of the container (2).

14. Method according to one of Claims 1 to 11, **characterized in that**
- for carrying out the plasma sterilization, the containers (2) are introduced into a chamber (3), into which a gas suitable for exciting a plasma is introduced, or remain in the said chamber, **in that**
- in the interior of the containers (2), an at least partial evacuation can be established via a feed line (7) shielded from the chamber (3), a gas pressure gradient being set and maintained in the interior in such a way that a plasma is excited there and maintained for a predetermined time, and **in that**
- the gas pressure gradient and the plasma in the interior of the containers (2) are maintained by an adequately low level of the pressure value in comparison with the pressure value in the chamber (3) even with a predetermined inflow of the gas from the chamber (3) into the containers (2) and subsequent suction removal from the containers (2).

15. Method according to Claim 14, **characterized in that**
- in a first method step, the chamber (3) is supplied with the gas, **in that**
- in a second method step, the containers (2) are evacuated to the extent that the excitation of the plasma in the interior takes place by means of the inflow of the gas from the chamber (3) and **in that**
- in a third method step, the gas supply into the chamber (3) is stopped, to excite a plasma in the chamber (3) and consequently on the outside of the containers (2) while at the same time extinguishing the plasma in the interior of the container (2).

16. Apparatus for carrying out the method according to one of the preceding claims, **characterized in that**
- the at least partly evacuable chamber (3) is a component part of a system (20) for producing and/or treating containers (2), in which a linkage of the production and/or treatment steps (21, 22, 23, 24) can be carried out in at least one process cycle.

17. Apparatus according to Claim 16, **characterized in that**
- in or on the chamber (3) there is an injection-moulding device, for producing at least one container (2).

18. Apparatus according to Claim 16 or 17, **characterized in that**
- in or on the chamber (3) there is a filling and/or closing device, by means of which the containers (2) can be filled with a medium and, if appropriate, can be closed and sealed.

19. Apparatus according to one of Claims 16 to 18, in particular for carrying out the method according to one of Claims 12 to 15, **characterized in that**
- in the chamber (3) there is at least one container holder, in which the containers (2) are held in a virtually pressure-tight manner with a predetermined leakage, **in that**
- at least one evacuation pump (9) and/or a gas supply (11) is connected to the chamber (3) for introducing the non-coating and/or coating gases and also a filling apparatus for the containers (2), and **in that**
- a plasma source (8) is attached to the outside of the chamber (3).

## Revendications

1. Procédé de traitement et/ou de fabrication de récipients en matière plastique (2), selon lequel
- dans au moins une étape du procédé, on effectue un traitement au plasma dans une chambre (3) en excitant une oscillation électromagnétique,
telle que le plasma, excité sous vide dans l'espace et/ou de manière sélective dans le temps s'applique aux parois du récipient (2), et
- en utilisant le vide on exécute d'autres étapes de fabrication et/ou de traitement (21, 22, 23, 24) sur ou avec les récipients en matière plastique (2) de façon à les enchaîner l'un à l'autre pendant le procédé de fabrication ou de traitement pour former un emballage stérile de produits dans les récipients en matière plastique (2).

2. Procédé selon la revendication 1,
**caractérisé en ce que**
le traitement au plasma est une stérilisation et/ou une dépyrogénéisation.

3. Procédé selon la revendication 1 ou 2,
**caractérisé en ce qu'**
une autre étape de traitement (22) consiste à réaliser un revêtement de couche barrière de perméation, de préférence des parois intérieures des récipients (2).

4. Procédé selon la revendication 3,
**caractérisé en ce que**
le revêtement se fait par application d'une couche de polymère par plasma.

5. Procédé selon la revendication 1 ou 2,
**caractérisé en ce qu'**
on modifie au moins des zones de la surface des récipients (2) chimiquement par un traitement au plasma de façon à rendre plus difficile la perméation de matière à travers la paroi des récipients.

6. Procédé selon l'une des revendications 3 à 5,
**caractérisé en ce qu'**
on effectue le traitement au plasma ainsi que le revêtement et/ou la modification de la surface des récipients (2) sous la forme d'au moins une autre étape de traitement dans un cycle de procédé exécuté dans la chambre.

7. Procédé selon l'une des revendications précédentes,
**caractérisé en ce que**
pendant ou après le traitement au plasma on effectue une activation au plasma (23), de préférence du côté extérieur des récipients (2).

8. Procédé selon l'une des revendications précédentes,
**caractérisé en ce qu'**
on réalise (21) les récipients (2) en amont ou dans la chambre, de préférence en utilisant le vide avant d'autres étapes de traitement (22, 23, 24).

9. Procédé selon la revendication 8,
**caractérisé en ce qu'**
on fabrique (21) les récipients (2) dans la chambre (3) par injection, emboutissage profond, soudage de films en matière plastique ou par extrusion, gonflage ou procédés analogues.

10. Procédé selon l'une des revendications précédentes,
**caractérisé en ce qu'**
on remplit les récipients (2) dans une autre étape de traitement (24).

11. Procédé selon l'une des revendications précédentes,
**caractérisé en ce qu'**
on ferme et/ou on scelle les récipients (2) dans une autre étape de traitement (24).

12. Procédé selon l'une des revendications précédentes,
**caractérisé en ce qu'**
- on dirige ou on laisse les récipients (2) pour exécuter le traitement au plasma dans la chambre (3) dans laquelle on peut réaliser pratiquement le vide dans des zones partielles,
- à l'intérieur des récipients (2), à l'aide d'une conduite d'alimentation (7) protégée par la chambre (3), on fait passer un gaz permettant d'exciter un plasma, le gradient de pression du gaz à l'intérieur étant réglé et maintenu pour y exciter un plasma et le maintenir pendant une durée prédéterminée, et
- on maintient le gradient de pression de gaz et le plasma à l'intérieur des récipients (2) à l'aide d'une pression d'amplitude suffisante par rapport à la pression dans la chambre (3) également avec une sortie prédéterminée du gaz de sortie des récipients (2) dans la chambre (3) et ensuite aspiration dans la chambre (3).

13. Procédé selon la revendication 12,
**caractérisé en ce que**
- dans une première étape de procédé on fait le vide dans la chambre (3),
- dans une seconde étape de procédé on fait arriver le gaz dans les récipients (2) pour exciter le plasma à l'intérieur,
- dans une troisième étape de procédé on fait arriver un gaz dans la chambre (3) pour exciter un plasma, et pour exciter le plasma à l'extérieur des récipients (2) en éteignant en même temps le plasma à l'intérieur d'un récipient (2).

14. Procédé selon l'une des revendications 1 à 11,
**caractérisé en ce qu'**
- on fait passer les récipients (2) dans une chambre (3) pour effectuer la stérilisation du plasma, où on les laisse séjourner dans cette chambre dans laquelle on fait arriver un gaz approprié pour exciter un plasma,
- à l'intérieur des récipients (2) on fait au moins partiellement le vide par une conduite (7) protégée par la chambre (3), en réglant un gradient de pression de gaz à l'intérieur et en le maintenant de façon à y exciter un plasma et à le maintenir pendant une durée prédéterminée, et
- on maintient le gradient de pression de gaz et le plasma à l'intérieur des récipients (2) par une diminution suffisante de la pression par rapport à celle dans la chambre (3) avec également une arrivée prédéterminée de gaz de la chambre (3) dans les récipients (2) suivie de l'aspiration dans les récipients (2).

15. Procédé selon la revendication 14,
**caractérisé en ce que**
- dans une première étape on alimente la chambre (3) en gaz,
- dans une seconde étape on fait le vide dans les récipients (2) pour que l'arrivée du gaz de la chambre (3) produise l'excitation du plasma à l'intérieur, et
- dans une troisième étape on arrête l'alimentation en gaz dans la chambre (3) pour exciter un plasma dans la chambre (3) et ainsi à l'extérieur des récipients (2) tout en éteignant le plasma à l'intérieur des récipients (2).

16. Dispositif pour la mise en oeuvre du procédé selon l'une des revendications précédentes,
**caractérisé en ce que**
la chambre (3) au moins partiellement mise sous vide fait partie d'une installation (20) pour fabriquer et/ou traiter des récipients (2) dans laquelle on effectue un chaînage des étapes de fabrication et/ou de traitement (21, 22, 23, 24) dans au moins un cycle de procédé.

17. Dispositif selon la revendication 16,
**caractérisé en ce que**
dans ou au niveau de la chambre (3) il y a une installation d'injection pour fabriquer au moins un récipient (2).

18. Dispositif selon la revendication 16 ou 17,
**caractérisé par**
une installation de remplissage et/ou de fermeture prévue dans ou sur la chambre (3) à l'aide de laquelle on peut remplir les récipients (2) avec un milieu et le cas échéant les fermer et les sceller.

19. Dispositif selon l'une des revendications 16 à 18, notamment pour la mise en oeuvre du procédé selon l'une des revendications 12 à 15,
**caractérisé en ce que**
- dans la chambre (3) il y a au moins un support de récipient sur lequel on tient les récipients (2) pratiquement de manière étanche à la pression et avec une fuite prédéfinie,
- on relie à la chambre (3) au moins une pompe à vide (9) et/ou une alimentation en gaz (11), pour fournir le gaz qui assure le revêtement et/ou n'assure pas le revêtement, ainsi qu'un dispositif de remplissage pour les récipients (2), et
- à l'extérieur de la chambre (3) on a une source de plasma (8).
